Europäisches Patentamt

**European Patent Office**

Office européen des brevets

(11) Numéro de publication: **0 029 411**
**B1**

(12) **FASCICULE DE BREVET EUROPEEN**

(45) Date de publication du fascicule du brevet:
**30.05.84**

(51) Int. Cl.³: **C 09 J 3/14, C 12 N 11/08**

(21) Numéro de dépôt: **80810349.3**

(22) Date de dépôt: **13.11.80**

(54) **Composition adhésive pour le dépôt d'un revêtement adhésif fixateur de molécules biofonctionnelles, revêtement obtenu et procédé pour sa préparation, substrat recouvert du revêtement et son utilisation comme biocatalyseur.**

(30) Priorité: **15.11.79 CH 10191/79**

(43) Date de publication de la demande:
**27.05.81 Bulletin 81/21**

(45) Mention de la délivrance du brevet:
**30.05.84 Bulletin 84/22**

(84) Etats contractants désignés:
**AT BE CH DE FR GB IT LI LU NL SE**

(56) Documents cités:
**DE - A - 2 237 083**
**FR - A - 2 187 849**
**FR - A - 2 281 377**
**FR - A - 2 281 968**
**FR - A - 2 281 969**

**JOURNAL OF THE AMERICAN CHEMICAL SOCIETY,**
vol. 100, no. 1, 1978, pages 302-304 A. POLLAK et al.:
*"Immobilization of synthetically useful enzymes by condensation polymerization"*

Le dossier contient des informations techniques présentées postérieurement au dépôt de la demande et ne figurant pas dans le présent fascicule.

(73) Titulaire: **BATTELLE MEMORIAL INSTITUTE, 7 route de Drize, CH-1227 Carouge/Genève (CH)**

(72) Inventeur: **Schneider, Michel, Domaine du Moulin 34, route d'Annecy, CH-1256 Troinex (CH)**
Inventeur: **Chevreux, Pierre, Résidence Prénépla, Ornex F-01210 Ferney-Voltaire (FR)**
Inventeur: **Guillot, Christian, Saint Nicolas le Vieux, 11, CH-1227 Carouge (CH)**

(74) Mandataire: **Dousse, Blasco et al, 7, route de Drize, CH-1227 Carouge/Genève (CH)**

# Description

### Domaine technique

La présente invention concerne le domaine des produits synthétiques biologiquement actifs et, plus particulièrement, celui des substrats dont la surface possède une activité biologique, notamment enzymatique ou autre.

Des travaux considérables ont été effectués ces dernières années pour mettre au point des matériaux, sous forme divisée ou non, dont la surface permet de fixer, de façon permanente ou temporaire, des molécules biologiquement actives. Par «molécules biologiquement actives» on entend, en général, des molécules qui ont un rôle à jouer dans les processus chimiques dont dépendent les êtres vivants. Parmi de telles substances on peut citer les enzymes, les inhibiteurs d'enzymes, les ferments, les hormones, les antigènes, les anticorps, les inhibiteurs biologiques, l'héparine, les lectines, etc... Ces molécules présentent généralement un ou plusieurs sites réactifs spécifique de telle ou telle réaction ou processus particulier et, par extension, on désignera également dans la présente description par le terme de molécules biologiques ou biofonctionnelles les substances d'origine synthétique ayant un comportement analogue, et pouvant fonctionner comme biocatalyseurs, biosorbants, etc...

Ainsi, de tels matériaux supports contenant une substance biofonctionnelle peuvent agir, vis-à-vis d'un substrat, comme le ferait la substance biofonctionnelle elle-même à l'ètat libre; cependant, à la fin de la réaction, de tels matériaux peuvent généralement être isolés du milieu réactionnel par les moyens habituels sans difficultés particulières alors que ce n'est pas nécessairement le cas pour une substance biologiquement active à l'état libre.

Un exemple particulièrement connu de tels matériaux biologiquement actifs est celui des résines recouvertes ou imprégnées d'une enzyme; ces matériaux, une fois activés, sont mis en présence d'un substrat à modifier biochimiquement de manière qu'une réaction enzymatique entre celui-ci et la couche active du matériau s'effectue.

Une fois la réaction terminée, le matériau actif est séparé du milieu de réaction par les moyens habituels et il peut être réutilisé généralement sans autre ou, si besoin est, son activité peut être restaurée par un traitement de régénération approprié. Ainsi, lorsque de telles substances biofonctionnelles sont fixées à un support inerte et que, dans cet état, elles peuvent agir sur un substrat à transformer, on les appelle souvent du nom de «biocatalyseur». De façon analogue une «biosorbant» est un substrat sur lequel on a fixé des molécules biofonctionnelles capable de capturer une substance que l'on désire séparer d'un mélange.

### Etat de la technique

Parmi les publications récentes les plus marquantes dans ce domaine on peut citer, à titre d'exemple, les documents suivants:

1. Pellicular Immobilized enzymes par C. Horwath, Biochim. & Biophys. Acta 338 (1974), 164–177. Cette référence souligne les avantages résultant de l'utilisation de pellicules minces d'un substrat contenant une enzyme par rapport à l'utilisation d'une enceinte remplie de particules d'un tel substrat à l'état divisé, par exemple colonnes chargées de particules de résine contenant des enzymes immobilisées dans la masse de celle-ci. En effet, l'action d'un tel substrat sur une solution avec laquelle il est en contact est essentiellement de nature superficielle et l'emploi de pellicules minces de matière active déposée sur un support rigide est avantageux pour des raisons évidentes d'économie de matière et de stabilité mécanique. Cette référence indique, notamment, que les matières en couches minces déposées sur un support rigide, une bille de verre par exemple, activées par des enzymes peuvent être classées dans trois catégories principales, à savoir:

a) l'enzyme est immobilisée dans une matrice à consistance de gel, l'enzyme étant liée au gel avant ou après le dépôt de celui-ci sur le support.

b) Le substrat consite en une couche microporeuse formée, par exemple, d'un absorbant minéral ou organique (silice, alumine, carbone, nylon poreux, etc...) et l'enzyme est fixée sur ce substrat par couplage ou par réticulation à la surface de celui-ci, (voir également USP 4 025 669).

c) Le substrat pelliculaire minéral organique est macroporeux (pores de l'ordre de 100–200 nm) il est possible de l'imprégner par une solution d'un couple («conjugate») enzyme-polymère après quoi on fait évaporer ce solvant de façon que ledit couple se dépose sur le substrat. Dans un tel cas, le système comporte donc, dans son ensemble, trois phases distinctes: (i) un support interte (bille de verre) recouvert (ii) d'un premier substrat (par exemple, polymère inerte macroporeux), la surface des pores de celui-ci étant elle-même (iii) recouverte d'une couche d'un autre polymère contenant l'enzyme.

Par ailleurs, la même référence décrit, dans le cadre des matériaux de la catégorie (b) ci-dessus, une méthode pour revêtir des billes de verre au moyen d'une solution d'un polymère riche en groupe –COOH (un copolymère d'anhydride maléique et d'éther méthyl-vinylique) et, après séchage, pour activer cette pellicule de polymère par contact avec des solutions aqueuses d'enzymes telles que trypsine, chymotrypsine, protéase, ribonucléase, papaïne, etc...

2. Les brevets français Nos 2 028 702 et 2 035 774 décrivent la fixation de molécules biologiquement actives, telles que des enzymes, antigènes, allergènes, hormones, etc., sur des supports organiques ou minéraux tels que cellulose, amidon, alginates, collagène, polysilanes, résines polyacryliques, verre, métal, etc., en utilisant des agents de pontage, tels que composés diazo, époxides, carbodiimides, chlorure de sulfonyle et dialdéhydes, le glutaraldéhyde par exemple. Le glutaraldéhyde agit également comme réticulant de telles molécules biologiquement actives.

Une méthode similaire est aussi décrite dans l'article suivant: Preparation and properties of Urease Chemically Attached to Nylon Tubes par P.V. Sundaram et al, Febs letters 10 5, 325 (1970), ainsi que dans la publication de demande de brevet allemand DOS 2 636 206.

3. Le brevet d'Allemagne de l'Est N° 106 856 (CA 82, 82 332q) décrit la fabrication d'un gel de poly-acroléine dans lequel on a incorporé, par mélange, de l'uréase.

4. Le brevet USP 3 844 892 (CA 82, 83 328t) décrit l'immobilisation de la glucose oxidase par réaction avec un copolymère époxydique obtenu par copolymérisation de nitriles acryliques et d'esters époxyacryliques. La fixation de l'enzyme se fait donc là par réaction de celle-ci avec le groupe oxirane du copolymère.

5. La demande de brevet japonais Kokai 75/10.585 (CA 83, 159 962 a) décrit la polymérisation d'un mélange d'asparaginase, d'acrylamide, de stabilisateur et d'initiateur de polymérisation, le tout déposé sous forme d'une couche mince, sur la paroi d'un tube de verre.

6. La demande de brevet japonais Kokai 75/70.584 (CA 83, 159 963 b) décrit également un film de polymère dans lequel est noyée une enzyme. Ce polymère est constitué d'un mélange de l'enzyme (aminoacylase) avec de l'acrylamide, du N,N'-méthylène-bis-acrylamide, du β-aminopropionitrile et du $K_2S_2O_7$ qu'on a déposé et fait polymériser sur un support hydrophile fritté.

7. La demande de brevet japonais Kokai 76/63.985 (CA 85, 107 494 w) décrit la formation d'un copolymère similaire à celui mentionné au point (5) ci-dessus et son utilisation pour décomposer l'acide aspartique d'une solution sanguine qu'on fait circuler au contact de ce copolymère actif.

8. La demande de brevet japonais Kokai 77/145.592 (CA 88, 117 050 z) décrit la préparation d'un film de nitrocellulose contenant une enzyme, ce film étant obtenu par adjonction de l'enzyme à une solution de polymère dans un solvant organique, puis dépôt du mélange sur un support réticulé en nylon et séchage du revêtement ainsi obtenu.

Certaines méthodes font appel à des processus de photopolymérisation; ainsi:

9. La demande de brevet allemand DOS 2 305 320 (CA 80, 24 327 q) décrit l'incorporation d'enzyme (urease) à du poly(2-hydroxyéthyl)-métacrylate puis de dépôt de ce mélange sur un support de polyéthylène, le durcissement de ce dépôt par irradiation (UV) et la protection de ce dépôt par une nouvelle couche de poly-(2-hydroxyéthyl)-méthacrylate de manière à fournir un film enzymatiquement actif et stable au stockage.

10. A. Tanaka et al, J. Ferment. Technol. 1977, 55 (1), 71–5 dédrivent le piègeage d'enzymes ou de cellules microbiennes par des résines photoréticulables, telles que le diméthacrylate de polyéthylène glycol.

11. La demande de brevet japonais Kokai 77/151.788 (CA 88, 117 046 c) décrit le piègeage d'enzymes dans une dispersion de résine photodurcissable. Suivant cette référence, on mélange une résine obtenue à partir de polyéthylèneglycol, de 2-hydroxyéthylméthacrylate et de xylène diisocyanate avec une poudre de diatomées et un photoinitiateur, puis on ajoute une solution aqueuse d'enzyme (invertase) et on irradie le mélange au moyen d'une lampe UV de manière à obtenir une masse enzymatiquement active.

12. La demande de brevet japonais Kokai 77/143.279 décrit la mise en suspension d'une protéine biologiquement active dans une résine photosensible liquide contenant un photosensibilisateur hydrosoluble, le tout étant ensuite émulsifié dans une phase aqueuse et irradié de manière à réaliser le piègeage («immobilisation») de ladite protéine par photopolymérisation des particules de résine contenant celle-ci.

13. La demande de brevet japonais Kokai 77/143.282 (CA 88, 117 048 e) décrit une méthode suivant laquelle on irradie, par rayonnement UV, un mélange d'une enzyme et d'une résine photopolymérisable, ce mélange étant préalablement utilisé pour imprégner un support poreux, par exemple un réseau de fils textiles, le tout formant alors une bande souple recouverte d'une pellicule enzymatiquement active.

14. Dans la demande de brevet japonais Kokai 75/78.641 (CA 84, 3309 c), on décrit le mélange d'une enzyme en solution aqueuse (glucose isomérase) et d'un monomère acrylique photopolymérisable puis la transformation, par irradiation UV de ce mélange, en une poudre de polymère contenant, «immobilisée», l'enzyme sous forme active.

15. Dans la demande de brevet japonais Kokai 78/34.984 (CA 89, 19 234 p), on décrit une méthode suivant laquelle on mélange une enzyme (β-Glucosidase) avec une solution aqueuse de gélatine et d'un monomère photoréticulant fixateur de l'enzyme (le 4,4'-diazidostilbène-2,2'-disulfonate), puis on dépose un film de ce mélange (50 m) sur une plaque de verre et on irradie le tout afin de préparer un film enzymatiquement actif.

Les référence suivantes concernent la préparation de matériaux de forme divisée dont les particules sont recouvertes d'une préparation biologiquement active, les molécules actives étant généralement fixées au substrat par pontage.

16. Le brevet USP 4 070 246 (CA 88, 117 052 b 1978) décrit un procédé suivant lequel on met en suspension une poudre métallique dans un mélange aqueux d'acide p-aminobenzoïque et de formaldéhyde, après quoi on filtre la poudre enrobée du polymère résultant de la condensation de ces composés, on soumet celle-ci à une diazotation et on fait copuler le substrat ainsi diazoté avec une enzyme de manière à fixer celle-ci sur le polymère.

17. Dans le brevet USP 4 071 409 (CA 88, 117 053 c 1978), on décrit le traitement d'un substrat tel que la silice ou le rutile en poudre par un di-isocyanate, ce dernier servant en l'occurence de pont pour la fixation, sur le substrat, d'une enzyme telle que la papaïne.

18. Le brevet USP 4 072 566 (CA 88, 117 054 d) divulgue une méthode suivant laquelle on adsorbe de la p-phénylènediamine sur des particules de verre poreux, on diazote l'amine aromatique et on fait copuler le produit diazoté avec de la papaïne.

19. Le brevet USP 4 007 089 (CA 86, 116 834 a) décrit l'utilisation de 1-N(2-nitro-4-azidophényl)-6-N'(4,6-dichloro-triazinyl)-diaminohexane pour ponter des enzymes et des anticorps sur des supports organiques non solubles dans l'eau, le groupement azido se transformant en groupe nitrène par irradiation UV et se fixant au support tandis que le groupe triazinyl se lie à la molécule protéinique enzymatique.

20. La demande de brevet japonais Kokai 76/31.689 (CA 85, 47 837 y) décrit le traitement de substrat hydroxylés (par exemple silicagel) par des aminosilanes et des polymères contenant des groupes dinitrobenzène-pyridinium afin de rendre ces substrats aptes à fixer des enzymes de façon stable.

21. Le brevet français N° 2 235 133 (CA 83, 74 687q) décrit la fixation d'enzymes (aminotransférase) sur des films ou fibres de collagène par activation préalable au HCl puis pontage par traitement au moyen d'hydrazine en présence d'acide nitreux.

22. La demande de brevet allemand DOS 2 323 422 décrit la préparation de copolymères de méthacrylate de 2-hydroxyéthyle et de diméthacrylate d'éthylène-glycol contenant, liés de manière covalente, des molécules biologiquement actives telles que, respectivement, des inhibiteurs d'enzymes, des enzymes, des antigènes, des anticorps, etc... Des résines particulaires constituées par de tels polymères fournissent des charges actives permettant de fixer, par contact avec leurs solutions, les substances conjuguées de celles que sont fixées sur ledit polymère, à savoir, respectivement, des enzymes, des inhibiteurs d'enzymes, des anticorps, des antigènes, etc...

Outre les pontages chimiques réalisés par les méthodes mentionnées ci-dessus, on peut citer la fixation des enzymes sur des supports par des méthodes de greffage faisant intervenir des radiations plus ou moins énergétiques. Ainsi:

23. H. Barker et al, Proc. Int. Congr. Catal., 6th 1976, Publ. Chem. Soc. Letchworth, Engl., (CA 87, 196 426 g) décrivent le greffage sous irradiation de monomères, tels que le p-nitrostyrène, sur des supports tels que les polyoléfines et le PVC, le groupe NO$_2$ étant ensuite converti en isothiocyanate, un groupe fixateur d'enzymes (trypsine). Comme type possible de sources d'irradiation, la référence cite les rayons γ, un faisceau d'élection et les UV.

24. Dans l'article de A.S. Hoffman et al, Trans., Am. Soc. Artif. Intern. Organs. 1972, 18, 10–18, il est indiqué qu'on a pu greffer, par irradiation γ sur des supports de caoutchouc silicone, des hydrogels de photopolymères contenant du méthacrylate de 2-hydroxyethyle et de la N-vinylpyrrolidone, ce greffage étant réalisé directement ou par l'intermédiaire d'acide ε-aminocaproïque.

Ces hydrogels ont ensuite permis de fixer chimiquement des molécules biologiques telles que l'albumine, l'héparine et la streptokinase, le tout constituant des substrats biologiquement actifs.

25. De même, A.S. Hoffman et al dans Am. Chem. Soc. Div. Org. Coat. Plast. Chem. Pap. 1974, 34 [1], 568–73 passent en revue les méthodes connues permettant de fixer, par les techniques d'irradiation, des polymères hydrophiles sur des supports inertes, des molécules biologiquement actives pouvant ensuite être fixées chimiquement sur les groupements –OH, –COOH, etc. libres de ces polymères hydrophiles.

26. De telles méthodes sont également décrites par A.S. Hoffman dans les références suivantes:
Polym. Prepr. Amer. Chem. Soc., Div. Polym. Chem. 1972, 13 [2], 740–6 et 723–8; USP 3 826 678.

Un perfectionnement dans la réalisation de polymères aptes à fixer des molécules biologiquement actives consiste, non pas à immobiliser celles-ci par piégeage dans la masse du polymère ou à les greffer sur un substrat donné, mais bien à préparer un copolymère entre un monomère de base assurant la formation de l'assise proprement dite du copolymère et un autre monomère lié à une molécule biologiquement active ou porteur d'un groupe réactif apte à fixer, ultérieurement, une molécule biologiquement active, cette liaison étant réalisée, soit directement, soit par l'intermédiaire d'une chaîne additionnelle (spacer) permettant de maintenir la molécule active à une distance choisie de substrat; cette distance est généralement adaptée de manière que les déplacements des portions de molécules mises en jeu pendant la réaction biologique ne soient pas entravés par la trop grande proximité de ce substrat.

27. Le brevet français 2 212 340 décrit ainsi une méthode suivant laquelle on polymérise ou copolymérise un monomère porteur d'une enzyme en présence d'un réticulant et d'un initiateur, l'enzyme fixée étant, par exemple, l'uréase, l'amyloglucosidase, la glucose oxidase, la fumarase, l'aspartase et la pénicelline aspartase.

28. Dans la demande de brevet allemand DOS 2 426 988, on décrit la fixation d'enzymes à des substrats par liaison covalente en faisant copolymériser un polysaccharide activé et un monomère, celui-ci possédant un groupement susceptible de se lier à une enzyme. On décrit également, en variante, la fixation d'une enzyme à un copolymère par réticulation de celui-ci au moyen d'un agent réticulant auquel on a préalablement fixé ladite enzyme.

Il est utile à ce stade de donner quelques précisions sur les applications des substrats biologiquement actifs, notamment lorsque ceux-ci se présentent sous formes de couches minces sur des supports minéraux ou organiques. Une des applications majeures de tels substrats est constituée par l'analyse automatique de certains fluides biologiques dont un exemple type est représenté par le dosage de glucose dans le sang et le sérum. L'échantillon à analyser est mis en circulation, après dilution appropriée et dialyse éventuelle, dans un circuit comprenant, en tant qu'élément réactif, un tube ou une batterie de tubes dont la paroi interne est recouverte d'un revêtement enzymatiquement actif vis-à-vis de la solution à analyser. A la sortie dudit élément, les produits de la réaction entre la solution et le revêtement sont analysés par les méthodes habituelles, par exemple par spectrophotométrie en continu. L'élément actif d'un tel dispositif d'analyse doit donc conserver intactes, avec le temps, ses propriétés enzymatiques et, de plus, posséder une spécificité et une stabilité suffisantes pour garantir la reproductibilité des résultats au cours d'une période prolongée de travail. Par ailleurs, l'activité spécifique du revêtement actif doit être élevée pour que l'élément actif ne soit pas trop encombrant au point de vue de ses dimensions. L'état de la technique dans ce domaine peut être illustré par les références suivantes:

29. Open Tubular Heterogeneous Enzyme Reactors: C. Horwath et B.A. Solomon, Biotechnology & Bioengineering 14, 885–914 (1972).

30. L.P. Leon & al, Clin. Chem. 22/7, 1017–1023 (1976); 23/9, 1556–1562 (1977).

Les référence citées dans le rapport de recherche sont les suivantes:

Les documents FR-A-2 281 968 et 2 281 969 mentionnés par ailleurs dans la présente description; le document DE-A-2 237 083 décrit, de manière générale, des monomères polymérisables ou copolymérisables (pouvant comporter, comme fonction polymérisable, une double liaison) dont une partie de la molécule comporte un site utilisable pour fixer des substances biofonctionnelles, par exemple, des enzymes. Le document «Journal of the Chemical Society», 100, N° 1 (1978) p. 302–304, intitulé «Immobilization of Synthetically Useful Enzymes by Polymerization Condensation» décrit la préparation d'un copolymère d'acrylamide et de N-acryl-succinimide en présence d'un initiateur de radicaux libres. Ce copolymère, sous forme de gel, sert alors pour immobiliser des enzymes suivant les moyens traditionnels déjà évoqués dans la présente demande.

Au vu des références précitées, on peut conclure que, à l'heure actuelle, la technologie des revêtements enzymatiquement actifs en couches minces sur des supports rigides se résume aux procédés suivants:

A. Gels contenant, immobilisée, la molécule biofonctionnelle, ces gels étant déposés, sous forme d'un revêtement sur un support.

B. Substrats poreux déposés, sous forme d'une couche mince, sur un support et contenant l'enzyme à l'état adsorbé ou fixée de manière covalente.

C. Support dont la surface elle-même (généralement lisse ou rugueuse) a été activée, soit chimiquement soit par irradiation, de manière à devenir apte à fixer l'enzyme, soit directement soit par l'intermédiaire d'un agent de pontage.

D. Revêtement de résine polymère contenant l'enzyme, soit piégée dans la masse, soit fixée par liaison covalente.

Dans tous les cas, les problèmes principaux liés à l'obtention de couches d'une efficacité satisfaisante sont les suivants.

a) Surface de contact effective entre la solution à traiter et le substrat actif aussi grande que possible, d'où bonne pénétration de ladite solution dans le revêtement actif; porosité élevée des gels ou substrats poreux; faculté de gonflement des matrices de résines polymères; hydrophilie suffisante des polymères utilisés.

b) Spécificité déterminée dans la nature des sites récepteurs et fixateurs des molécules bioactives suivant les buts recherchés: obtention d'un substrat d'activité biologique permanente ou extraction temporaire de substance active avec relargage ultérieur (purification d'enzymes ou chromatographie d'affinité).

c) Possibilité d'utiliser la totalité ou presque de la masse active, certaines substances biofonctionnelles étant très onéreuses, d'où possibilité de produire des revêtements actifs dont seules les portions accessibles à la solutaion à traiter contiendront des molécules actives.

d) Adhésion irréprochable du substrat actif sur son support.

Or, à l'heure actuelle aucune des techniques connues ne permet de concilier simultanément ces exigences à un degré élevé. En effet, les gels et les substrats poreux sont difficiles à faire adhérer solidement à un support inerte et, par ailleurs, leur perméabilité et leur porosité sont difficiles à contrôler de manière à assurer une circulation libre et efficace entre les sites actifs et la solution à traiter.

D'autre part, le traitement direct des supports par voie chimique ou par irradiation manque souvent d'efficacité (taux de fixation superficiel insuffisant); de plus, les irradiations très énergiques (rayons $\gamma$) peuvent avoir un effet destructeur sur lesdits supports.

Par ailleurs, les méthodes actuelles manquent de versatilité en ce sens que chaque cas particulier réclame une technique individuelle souvent très différente de celle qu'exige un cas apparemment similaire et n'en différant que par un caractère d'aspect anodin. Ainsi, par exemple, il n'est pas possible, au moyen de techniques connues, de préparer une composition enzymatiquement active unique qui convienne à la fois au verre poreux et au poyéthylène lisse, c'est-à-dire qui puisse s'appliquer directement sur l'un de ces supports, au choix, sans traitement particulier propre à l'un ou l'autre de ces supports.

Description de l'invention

La présente invention remédie à ces inconvénients et met en œuvre une technique à la fois simple et d'une très grande versatilité en ce sens qu'elle s'applique pratiquement à tous les supports envisageables et que, par des modifications mineures et facilement prévisibles, elle peut s'appliquer à l'obtention de substrats biofonctionnels en couche mince d'une variété pratiquement illimitée.

Pour cela l'invention met en œuvre une composition photo-adhésive (I). Cette composition est adhésive pratiquement vis-à-vis de la totalité des supports solides minéraux et organiques usuels: verre, céramiques, métaux, bois, résines artificielles, etc... c'est-à-dire tous suports pouvant être raisonnablement envisagés pour une telle application et, cela sans qu'il soit nécessaire de procéder à un traitement particulier quelconque de la surface de ces supports. La composition de l'invention pour revêtements adhésifs de réserve hydrophile gonflant à l'eau et perméable à celle-ci est à base d'acide acrylique; elle contient, bien entendu, (i) au moins une cétone aromatique comme photo-initiateur et, en outre, (ii) un agent activateur de photopolymérisation (pour permettre une photo-adhésion et un photo-durcissement rapide) et promoteur d'adhésion (pour garantir une adhésion suffisante du revêtement photopolymérisé sur le support) constitué par un acrylate ou méthacrylate d'amino-alcool. La composition contient encore (iii) au moins un monomère comprenant un groupement apte à fixer, par liaison directe avec celui-ci ou par l'entremise d'un pont intermédiaire préalablement relié à ce groupement, une substance biologiquement active ou potentiellement biologiquement active. Le choix du monomère (iii) est mentionné plus loin.

On entend par «potentiellement biologiquement active» (par opposition à «directement ou cinétiquement active») une molécule normalement biofonctionnelle mais qui, dans certaines conditions, peut être inactivée, par exemple par couplage avec un inhibiteur ou par blocage de son ou ses sites bioactifs.

Lorsqu'on a appliqué la composition photo-adhésive de l'invention sur un support, on soumet le tout à une photo-irradiation, par exemple au moyen de rayons UV, ce qui provoque la copolymérisation de l'acide acrylique, du monomère (iii) et d'autres substances copolymérisables éventuellement encore présentes dans la composition. On obtient ainsi un revêtement photopolymérisé (II) de résine hydrophile adhérant fermement aux supports minéraux ou organiques synthétiques ou naturels, superficiellement perméable à l'eau et gonflant en présence de celle-ci, cette résine contenant, à l'état copolymérisé, au moins un type de maillon apte à fixer, par liaison directe ou par l'entremise d'un pont préalablement ou ultérieurement relié à celui-ci, une ou plusieurs substances macromoléculaires actives (ou potentiellement actives) et, cela, de façon stable et sans que l'activité réelle ou potentielle de ces substances soit sensiblement altérée ou modifiée. Bien entendu, un tel revêtement photopolymérisé (II) fait également partie de l'invention de même que le support revêtu d'un tel revêtement.

Une fois le revêtement photopolymérisé obtenu sur son support, on peut y fixer les substances biologiquement actives, soit par contact direct avec celles-ci, soit par fixation préalable d'un pont intermédiaire (pi), dont l'extrémité libre contiendra une fonction susceptible de fixer la substance biofonctionnelle, soit de manière permanente (covalence), soit de manière temporaire (complexation). Dans ce dernier cas, le pont intermédiaire sera considéré comme ligand spécifique de la molécule à capturer. Le revêtement photopolymérisé porteur de molécules biofonctionnelles (ou potentiellement biofonctionnelle) (III) fait également partie de l'invention, de même que le support recouvert d'un tel revêtement.

Modes de réalisation et mise en œuvre

Lorsque les molécules biofonctionnelles ont été liées sur le substrat de l'invention, on obtient ainsi un produit bioactif (ou potentiellement bioactif) qu'on peut mettre en œuvre dans les diverses applications auxquelles il est destiné. Par exemple, s'il s'agit d'un revêtement (III) porteur d'une enzyme, le substrat est mis en contact avec une solution de matières à traiter au moyen de cette enzyme, de manière que la réaction désirée s'effectue, en continu ou en discontinu. Puis on sépare le milieu réactionnel du substrat actif, on récupère celui-ci et on le réutilise autant de fois qu'on le désire. Suivant un autre exemple, s'il s'agit de la capture spécifique par complexation d'une molécule biofonctionnelle dans un mélange de celle-ci avec d'autres substances, on procède comme ci-dessus au moyen de substrat (II), puis on sépare le substrat (III) chargé de ladite molécule et, par un traitement approprié, on provoque la scission du complexe et la libération de la molécule capturée. Il est également entendu que la présente invention s'étend à l'utilisation des revêtements (II) et (III) comme agents actifs dans les réactions de transformation biochimiques propres aux substances biofonctionnelles fixées sur lesdits revêtements ou substrats. De tels ingrédients peuvent parfois être désignés sous le nom de «biocatalyseurs».

Comme on l'a vu plus haut, la composition (I) de l'invention est à base d'acide acrylique. Ce monomère convient particulièrement bien car il donne aux copolymères qu'on en obtient des propriétés hydrophiles favorables et, de plus, il polymérise facilement et rapidement sous l'influence de photo-initiateurs convenables. Par contre l'acide méthacrylique n'est pas à recommander pour la composition (I) car il ne photopolymérise pas assez rapidement.

Comme photo-initiateurs (i), on utilise, dans la composition (I) des cétones aromatiques habituellement utilisées comme photo-promoteurs de polymérisation (voir par exemple le brevet USP 3 759 807). On peut citer, par exemple, la benzophénone, l'acétophénone et les composés halogénés correspondants. On peut également utiliser des composés anthraquinoniques comme la 2-éthyl-anthraquinone, l'acide anthraquinone-2-carboxylique, de même que d'autres photo-initiateurs tels que la benzoïne.

Comme activateur de polymérisation et promoteur d'adhésion (ii), on utilise un acrylate ou méthacrylate d'amino-alcool tel que le méthacrylate de dimethylaminoéthyle (DMAEMA). Cependant, on peut également utiliser des métacrylates d'autres amino-alcools, tels que le N-diethyl- aminoéthanol ou le N-éthyl-N-ter.butylamino-éthanol. L'utilisation de tels activateurs dans les compositions d'adhésifs est d'ailleurs, en soi, connue (voir brevets français Nᵒˢ 2 881 968 et 2 281 969 décrivant des compositions photoadhésives à base d'acide acrylique et d'acrylates ou métacrylates de diacrylamino-alcools.

Outre l'acide acrylique en tant que monomère de base du présent copolymère, la composition peut encore contenir d'autres monomères oléfiniques tels que des esters acryliques et métacryliques, des acrylates polyfonctionnels et des prépolymères acryliques. Les esters acryliques monofonctionnels peuvent être ajoutés dans le but de donner plus de liant et une viscosité plus adéquate à la composition adhésive et, en outre, de régler l'hygrophilie du copolymère après photodurcissement. Les quantités de tels adjuvants sont donc à faire varier par l'homme de métier, suivant les besoins en fonction des propriétés à donner au copolymère envisagé. Parmi les esters qui peuvent être utilisés avantageusement, on citera les acrylates et méthacrylates d'alcoyles inférieurs (méthyle, éthyle, propyle, butyle, isobutyle, tert.butyle, etc...), de docécyle, d'éthyl-hexyle, de méthoxyéthyle, etc. Les acrylates polyfonctionnels peuvent être utilisés pour donner plus de rigidité au présent copolymère suivant les besoins. Comme tels, on peut citer le triacrylate de triméthylol-propane (TMPTA), le triacrylate de pentaerythrite (PETRA) et le tetraacrylate correspondant (PETEA). Les prépolymères acrylique ou vinyliques peuvent être ajoutés en petite quantité à la composition de l'invention lorsqu'on désire lui conférer plus de souplesse et d'élasticité. Comme tels prépolymères, on peut citer les composés connus sous les noms commerciaux de UVI-THANE (THIOKOL Corporation), EBECRYL (Union Chimique Belge). Par ailleurs, des polymères similaires sont décrits dans le brevet britannique Nᵒ 1 430 422 et la demande brevet allemand DOS 2 542 314.

Les monomères (iii) possédant une fonction réactive susceptible de lier, directement ou par l'intermédiaire d'un pont, des molécules biofonctionnelles sont extrêmement variés et peuvent être choisis, suivant les besoins, de cas en cas. De manière générale, il devront comporter une double liaison oléfinique acrylique ou vinylique et, lorsqu'il s'agit de fixer directement, par exemple, une grosse molécule peptidique (enzyme, inhibiteur d'enzyme, antigène, anticorps, etc...), un groupe fonctionnel susceptible de réagir avec, par exemple, un groupement amine de ladite molécule à capturer. Des composés contenant de tels groupes fonctionnels (agents acylants, groupes protecteurs, composés porteur de «leaving groups») sont abondamment décrits dans la littérature consacrée aux synthèses de polypeptides des monomères oléfiniques pourvus de tels groupes fonctionnels qui conviennent à la présente invention sont l'acrylate de N-hydroxysuccinimide, l'acrylamido-caproate de N-hydroxysuccinimide, l'acrylate d'époxypropanol et l'acrylate de 2-isocyanato-éthyle. De manière générale, d'autres monomères semblables, mais dans lesquels la double liaison et le groupe fonctionnel seront séparés par une chaîne hydrocarbonée d'une longueur supérieure à celle des monomères susmentionnés peuvent être avantageux dans le cas où on désire donner plus de liberté à la molécule biofonctionnelle, c'est-à-dire à la maintenir, pour des raisons d'encombrement stérique, à une distance plus grande du substrat. De manière générale une telle chaîne hydrocarbonée pourra avoir de 5 à 15 atomes de carbone.

Lorsqu'il s'agit de fixer des molécules bioactives sur le substrat par l'entremise du monomère (iii) et par l'intermédiaire d'un pont intercalaire (pi), ledit monomère (iii) comporte, outre la double liaison, un groupe réactif susceptible de former facilement, avec un composé approprié, ledit pont intercalaire. Comme tels groupes réactifs on cite premièrement les groupes $-OH$, esters, $-COOH$, $-NH_2$. En conséquence, comme monomères convenant à l'introduction de tels groupes, on peut citer le monoacrylate d'éthylène-glycol, l'acrylate du monoacétate de glycol, l'acrylate de 2-aminoéthanol, l'anhydride maléique et d'autres monomères similaires.

A titre d'exemple, on précisera que comme ponts intermédiaires (pi) on peut aussi utiliser des carbodiimides (réaction sur les groupes $-COOH$ avec formation d'acylurées, elles-mêmes réactives avec les molécules protéiniques); l'hydrazine réagissant sur les esters et formant, avec $HNO_2$, des groupes azides réactifs avec les enzymes); des diisocyanates (se liant, d'un côté sur les groupes $-OH$ [uréthannes] ou $-NH_2$ (urée) et, de l'autre, sur les molécules biofonctionnelles à piéger). De manière générale, lesdits ponts intercalaires comportent, à leur extrémité libre un groupement fonctionnel capable de réagir avec la molécule biofonctionnelle et de fixer celle-ci au substrat, la définition d'un tel groupement étant la même que celle donnée plus haut.

De manière plus détaillée, on peut encore ajouter les exemples suivants de ligands terminaux susceptibles de fonctionner comme bittes de fixation de molécules biofonctionnelles sur le revêtement de l'invention: l'aminobenzamidine spécifique de la trypsine; la 4-phénylbutylamine spécifique de la chymotrypsine; les colorants tels que le CIBACHRON BLUE F3G–A et le PROCION RED HE–3B spécifique des deshydrogénases; les sucres tels que le maltose qui est spécifique de certaines lectines ainsi que la D-galactosamine spécifique des galactosidases; l'acide iminodiacétique (IDA) qui, sous la forme de sel de Zn, complexe spécifiquement certaines protéines plasmatiques ($\gamma$-globulines, $\alpha_2$-macroglobulines, etc.), la lysine ainsi que le p-aminobenzoate de butyle qui sont spécifique du plasminogène. D'autres exemples figurent dans l'ouvrage «Methods in Enzymology, Vol. 34».

Les proportions des divers ingrédients que peut contenir la présente composition sont extrêmement variées et peuvent facilement être adaptées, suivant les besoins, par le technicien compétent. En règle générale, cependant, les proportions d'acide acrylique ne devraient pas être trop faibles pour que le polymère présente une hygrophilie suffisante et pourront être comprise entre environ 10 et 70% en poids de la composition et, de préférence, entre 20% et 50% en poids. Le photo-initiateur (i) est en concentration de 0,5 à 10%, de préférence aux alentours de 1 à 5% pour assurer une photopolymèrisation efficace et la proportion de l'activateur et promoteur (ii) est comprise entre environ 0,5 et 15%, la gamme préférée se situant entre 4 et 8% en poids. La quantité du monomère (iii) est essentiellement dépendante de l'activité spécifique qu'on désire impartir au copolymère une fois que celui-ci est déposé sur son support. Une telle activité commence à être significative lorsque la composition ne contient que des quantités relativement faibles de monomère (iii) mais, souvent, dans la pratique, on cherche à parvenir à des activités spécifiques (par unité de surface) aussi élevées que possible. Dans un tel cas, on incorpore à la composition jusqu'à 30 et même 50% du monomère (iii), la limite supérieure étant dictée par les propriétés physiques du revêtement obtenu par copolymérisation.

En effet, des quantités trop importantes du monomère (iii) peuvent engendrer l'apparition de défauts du revêtement: manque d'hydrophilie, manque d'adhésion, friabilité, etc...

Lorsque la présente composition contient, en plus, un ester acrylique, celui-ci pourra remplacer une partie de l'acide acrylique et sa concentration pourra être comprise entre 0 et 60% environ. Les autres acrylates (polyfonctionnels ou prépolymères) décrits plus haut seront éventuellement ajoutés en faibles quantités, ne dépassant pas, en général, 5 à 10%. Il reste cependant à noter que les concentrations ci-dessus ne sont pas critiques et peuvent être outrepassées, si besoin est, dans certains cas spéciaux.

En ce qui concerne la préparation de la présente composition adhésive, elle peut être réalisée facilement par mélange des divers ingrédients choisis, étant bien entendu que ceux-ci doivent être compatibles entre eux. Ce mélange peut être effectué par les moyens habituels, notamment au moyen d'un malaxeur. Une fois le mélange obtenu, on le laisse reposer quelques heures afin de la laisser s'équilibrer puis on peut le stocker à l'obscurité ou l'utiliser directement à la préparation de revêtements adhésifs. Pour préparer de tels revêtements, on dépose la composition sur un support suivant les techniques habituelles: pinceau, pulvérisation, lame d'étalement, etc... Comme on l'a déjà dit, pratiquement tous les supports conviennent: plaques, billes, tubes supports lisses ou rugueux, verre, métaux, plastiques, polyoléfines, nylon, PVC, résines expansées, etc... Dans la pratique, on préfère, par exemple, traiter des billes de verre ou en matière synthétique, de telles billes pouvant, par la suite, être utilisées comme bio-catalyseurs dans divers dispositifs de laboratoire ou industriels: colonnes de purification, enceintes de traitements de liquides biologiques, etc... On peut aussi recouvrir des films très minces de polyéthylène qui, une fois repliés ou enroulés, offrent une grande surface de contact dans un volume réduit; ou encore imprégner une mousse de polyuréthanne (transparente aux UV) de manière à obtenir une structure poreuse dont la surface interne, recouverte d'un film de la composition, est très étendue. Suivant une autre forme d'application, on pulvérise la composition sur la surface interne d'un tube, le revêtement ainsi obtenu servant, par la suite, d'élément d'analyse dans un appareil de mesure automatique de certains constituants de fluides biologiques.

La qualité de composition qu'on utilise par unité de surface du support peut être très faible. En effet, il a été constaté que lors du contact entre un fluide à traiter et la surface du revêtement, activé, obtenu à partir de la présente composition, seule une profondeur correspondant à quelques couches monomoléculaires était mise en jeu (10 à 20 nm environ). Aussi, la couche efficace des revêtements obtenus à partir de la présente composition peut-elle être très mince (de l'ordre de 0,1 à 5 g/m²) ce qui constitue un avantage économique non négligeable.

Une fois la présente composition étalée en couche sur tout ou partie du substrat porteur, on procède à son durcissement par photo-irradiation. Lorsque le support est transparent celle-ci peut se faire recto ou, de préférence, verso, l'adhésion étant améliorée dans ce cas.

Comme source de rayonnement, on peut utiliser toute source de rayonnement électromagnétique dont le spectre d'émission est constitué au moins en majeure partie par le domaine spectral situé au-dessus de 0,3 micron, par exemple une lampe à vapeur de mercure. On utilise, de préférence, une ou plusieurs lampes à vapeur de mercure ayant une puissance comprise entre 20 watts et 10 kilowatts.

Par exemple, on peut utiliser une lampe à vapeur de mercure ayant une puissance nominale de 2 kilowatts comme une lampe marque Philips vendue sous le désignation HTQ7 ou encore une lampe à vapeur de mercure ayant une puissance nominale de 5 kilowatts comme une lampe à vapeur de mercure à haute pression délivrant une énergie de 80 watts/cm, de marque Hanovia. Des lampes à argon ou à krypton peuvent également convenir.

La durée d'irradiation nécessaire pour obtenir la polymérisation de l'adhésif correspond à l'obtention d'une dose minimale d'irradiation de longueur d'onde appropriée reçue par la couche d'adhésif. Elle varie donc essentiellement en fonction de la puissance, de la distribution spectrale de la source de rayonmement et de la distance entre cette source et l'adhésif.

Cependant, la mise en œuvre de la composition selon l'invention permet d'obtenir avec des durées d'irradiations très courtes (comprises, par exemple, entre 0,5 seconde et quelques minutes suivant la puissance de la source de rayonnement utilisée et ses caractéristiques d'émission spectrale) une polymérisation et une adhésion satisfaisantes sur la presque totalité des supports envisageables. De plus, cette adhésion n'est que peu ou pas affectée lorsque le substrat, activé, est mis en contact avec les solutions aqueuses, notamment les solutions avec lesquelles on le fait réagir.

Une fois le revêtement photopolymérisé (II) obtenu, on procède à l'activation de celui-ci. Pour cela, il faut considérer, soit l'activation directe par mise en contact du substrat avec la ou les molécules biofonctionnelles à lier, soit l'activation par l'intermédiaire du pont intercalaire évoqué plus haut.

Dans le premier cas, on procède de manière très simple, généralement en immergeant le support muni du substrat réactif dans une solution de la substance à fixer et en l'y laissant, sous agitation, le temps voulu pour atteindre un taux de fixation suffisant. Dans la pratique, on trempe le substrat dans une solution aqueuse, par exemple d'une enzyme, et on laisse ces éléments en contact de quelques minutes à plusieurs heures, après quoi on lave le substrat activé jusqu'à disparition, dans les eaux de lavage, de l'enzyme libre.

Dans le second cas, on commence par procéder à la fixation du pont intercalaire, cela suivant les méthodes déjà évoquées plus haut ou suivant les méthodes abondamment décrites dans les références citées dans l'introduction. Puis on procède à la fixation des molécules biofonctionnelles comme décrit plus haut. Dans les exemples qui sont donnés plus loin on donne des précisions sur la manière de réaliser plusieurs formes d'exécution de l'invention.

En résumé, la présente invention présente, sur les techniques de l'art antérieur, les avantages suivants:

Mise en œuvre très simple et très rapide. Degré de spécificité des sites fixateurs très élevé et excellent contrôle du taux d'activation et de la nature des molécules fixées. Extrême versatilité de la méthode, sur la base d'une composition adhésive commune, par simple variation de la nature du monomère à copolymériser. Structure simple et bien définie du polymère, ainsi que du catalyseur biologique qu'on en obtient. Degré d'hygrophilie et perméabilité du polymère réglable à volonté. Adaptation à, pratiquement, tous les supports.

Suivant un exemple d'application typique d'un substrat activé (III) suivant l'invention, on revêt par pulvérisation la paroi interne de tubes de verre d'une couche d'environ 1 µm d'une composition (I) contenant, comme monomère (iii), de l'acrylate de N-hydroxysuccinimide et on irradie 10 sec au moyen d'une source UV de 2 kW placée à 15–20 cm du tube. Puis on active une portion du revêtement par immersion de la première moitié du tube dans une solution aqueuse de glucose oxydase et une seconde portion dudit revêtement par immersion de la seconde moitié du tube dans une solution de peroxydase. Le tube ainsi activé est ensuite disposé, comme élément actif, dans un appareil de mesure automatique de glucose dans un fluide biologique, la solution traversant le tube dans le sens glucose oxydase → peroxydase. Lors de l'analyse, le glucose contenu dans la solution passant dans la première moitié du tube est décomposé en présence de glucose oxydase avec libération de $H_2O_2$ et celle-ci est mise en évidence, par son action oxydante sur un indicateur (p-tolidine, coloration bleue), cette action étant catalysée par la peroxydase de la seconde moitié du tube actif.

Une méthode voisine permet de déterminer la quantité d'antigène d'un fluide biologique, ceci, en raison du degré de reproductibilité et fiabilité élevé de l'activité spécifique des revêtements suivant l'invention. On procède comme suit: on prépare un revêtement d'activité spécifique contrôlée en pulvérisant, à l'intérieur d'un tube donné, une quantité connue de composition et, après irradiation, on active le revêtement ainsi obtenu à un taux déterminé et reproductible par contact avec une solution d'anticorps. Puis, on mélange à la solution à analyser une proportion connue d'antigène marqué (+) (radioactif) et on met celle-ci en contact avec le substrat activé le temps voulu pour saturer les sites actifs de ce dernier. On mesure alors le taux de radioactivité de la solution résiduelle ce qui permet, par soustraction, de déterminer la quantité d'antigène marqué capturé et, par cela et en fonction de la quantité théorique devant être absorbé par le revêtement, la proportion d'antigène non marqué originalement présente dans l'échantillon.

Les exemples suivants illustrent l'invention en détail.

Applications de laboratoire et industrielles

Exemple 1

Préparation d'une composition adhésive (I)

Dans un malaxeur, on a mélangé intimement les ingrédients suivants: Acide acrylique 58,4%, méthacrylate de 2(diméthylamino)-éthyle (DMAEMA) 13%, acrylate de N-hydroxysuccinimide 26%, benzophénone 2,6%.

On a laissé reposer ce mélange plusieurs heures à température ambiante, puis on a étalé une couche mince de la composition sur une plaquette de verre surfacé au moyen d'un outil de couchage manuel (Hand Coater) puis, pour abriter l'enduit de l'air ambiant, on a appliqué sur celui-ci une feuille de polyester non traité qu'on a étalée, sans la presser, au moyen d'un rouleau de caoutchouc.

Irradiation et préparation du revêtement (II)

On a soumis la plaque de verre ainsi préparée à 2 min d'irradiation sous une lampe Philips (type HTQ7, 28 W/cm) placée à 15 cm du substrat. Puis on a détaché la feuille de polyester qui s'est séparée facilement. Le revêtement ainsi obtenu était incolore, ferme et non cassant; son épaisseur était d'approximativement 8 µm.

Activation et préparation du substrat activé (III) On a plongé la plaquette dans une solution aqueuse de 100 mg/ml de trypsine (SIGMA) à pH 7,5 (tampon phosphate 0,5 M) et on l'a laissé immergée 4 h dans cette solution à température ambiante. Puis on l'en a retirée et on a constaté que le revêtement avait sensiblement gonflé mais qu'il adhérait toujours parfaitement à son support. On a soigneusement lavé la plaquette au tampon phosphate 0,5 M puis on a déterminé l'activité spécifique du substrat en la plongeant dans une solution aqueuse $10^{-3}$ M de p-nitroanilide de benzoyl-arginine (BAPNA). Sous l'action enzymatique de la plaquette activée, ce composé a libéré de la p-nitroaniline qu'on a dosée spectrophotomètriquement à 410 nm. La vitesse d'hydrolyse enzymatique de ce substrat en solution était de 22 µmole/min à température ordinaire.

On en a déduit que l'activité spécifique du revêtement actif était de 50 µg de trypsine/cm².

Exemple 2

On a procédé comme décrit à l'exemple 1 et on a préparé une composition (I) photo-adhésive à partir de:

Acide acrylique 32,5%; acrylate de butyle 32,5%; DMAEMA 6,5%; acrylamidocaproate de N-hydroxysuccinimide 26%; benzophénone 2,5%.

On a appliqué cette composition (stockée vers 40 °C de manière à empêcher sa cristallisation par refroidissement) à l'intérieur d'un tube de polyamide 6.6 (Cellpack A.G., CH-5610, Wohlen, AG) de dimensions suivantes: longueur utile 5 cm; diamètre 0,15 cm; surface intérieure utile 2,35 cm². Pour ce faire, on a d'abord rempli le tube de la composition adhésive, on l'a laissé se vider par gravité puis on a chassé l'excès de liquide au moyen d'un jet d'air comprimé qu'on a fait circuler 3–5 sec dans le tube. Puis on a fait circuler un lent courant d'azote dans le tube tout en le soumettant 6 min à l'action d'une lampe UV de 2 kW placée à 15 cm et en le faisant lentement tourner axialement sur lui-même pendant le temps d'irradiation. Puis, comme à l'exemple précédent, on a immergé le tube ainsi préparé (phase II) dans une solution aqueuse de trypsine et, après 4 h à température ambiante, on a constaté que l'activité spécifique du revêtement (phase III) était de 36 µg de trypsine par cm².

Une série de 12 tubes similaires a été préparée de la même manière et on a constaté que, a peu de chose près, l'activité spécifique de chacun de ceux-ci était identique. Ces tubes ont été groupés en un faisceau et réunis dans un tube de verre de diamètre d'environ 1,2 cm de manière à constituer un élément actif dont l'activité totale était d'environ 1000 µg de trypsine.

Exemple 3

On a procédé comme décrit aux exemples précédents et on a préparé une composition adhésive contenant: acide acrylique 35%, acrylate d'isopropyle 30%; DMAEMA 6,5%; acrylate de 2-hydroxyéthyle 26%; benzophénone 2,5%.

On a dilué 100 g de cette composition dans 100 ml de méthyl-éthyl-cétone et on a pulvérisé ce mélange sous forme d'un brouillard très fin à l'intérieur de tubes de polyamide (de même type qu'à l'exemple 2) de manière à déposer, à la surface de ceux-ci, un film d'environ 1 µm. Puis on a rapidement séché les tubes sous courant d'azote, après quoi on a irradié le revêtement comme décrit dans l'exemple 2. Puis on a immergé ces tubes 15 min à température ordinaire dans une solution à 60% de butylène disocyanate

dans du CHCl₃ après quoi on a rincé les tubes au chloroforme de manière à éliminer l'excès de diisocyanate et on les a rapidement séchés sous N₂.

Pour rendre le revêtement biofonctionnel, on a procédé comme suit: on a disposé deux tubes en série et on y a fait circuler 2 h à température ambiante à la cadence de 1 ml/min 10 ml d'une solution à 1,5% d'héparine (SIGMA, 170 unités USP/mg). Puis on a lavé les tubes par une solution de NaCl à 0,9% et on y a fait circuler à 37 °C une solution constituée d'un mélange de 0,3 ml de plasma, 0,3 ml d'une solution de thrombine (~ 2,4 unités NIH [National Institute of Health, USA]) et 0,3 ml de solution physiologique. Après 1 min de circulation, on a déterminé le temps de coagulation de cette solution suivant la technique standard, la valeur trouvée étant de 44 secondes. A titre comparatif, on a fait circuler un échantillon témoin de la solution ci-dessus dans un jeu de deux tubes identiques, placés en série, mais dont le revêtement n'avait pas été activé à l'héparine et on a mesuré, alors, un temps de coagulation de 18 sec. L'héparine s'est donc bien fixée au revêtement suivant l'invention et le tube ainsi préparé présentait une action anticoagulante significative.

Exemple 4

On a utilisé la même composition adhésive que celle décrite à l'exemple 2 et, au moyen d'un dispositif de couchage à lame traînante, on a déposé un film d'environ 2–3 µm sur une feuille de polypropylène biorienté de 12 µm d'épaisseur.

On a fait circuler cette feuille, sous protection d'un courant d'azote projeté, à sa surface, sous forme d'un flux laminaire, en regard d'une lampe UV de 28 W/cm, de manière que chaque portion de cette feuille soit irradiée 3 sec à und distance de 8 cm. Puis on a découpé la feuille en bandes de 5 × 10 cm et on a enroulé ces bandes en spirale dans le sens de la longueur de manière à obtenir des cylindres qu'on a ensuite introduit dans des tubes de polyamide semblable à celui décrit dans l'exemple 2. Une fois le cylindre introduit dans le tube, la spirale se détend de manière que soit ménagé, entre les spires, un passage libre d'environ 2 à 5 µm d'épaisseur.

On a immergé alors les tubes dans une solution aqueuse de trypsine et, après avoir procédé comme décrit dans l'exemple 2, on a obtenu un substrat activé dont l'activité spécifique était d'environ 30 µg de trypsine/cm². On a alors réuni les tubes par faisceaux de 12 comme décrit à l'exemple 2 ce qui a permis d'obtenir des éléments actifs d'environ 6 cm³ dont la surface active totale était de 600 cm² et l'activité d'environ 18 mg de trypsine. On a testé cet élément en y faisant circuler à la cadence de 5 ml/min une solution $10^{-3}$M d'ester éthylique de la benzoylarginine (BAEE) ce qui a provoqué la libération pratiquement quantitative de la benzoylarginine (déterminée par titration suivant les moyens habituels).

Exemple 5

On a préparé une composition adhésive identique à celle de l'exemple 2, mais en remplaçant le monomère réactif de celle-ci par de la N-acryl-oyl-N'-t.butoxycarbonylhydrazine de formule $CH_2 = CH-CO-NH-NH-CO-O-t.Bu$. On a déposé cette composition sur une feuille de polypropylène comme décrit dans l'exemple précédent et, après irradiation, on a préparé des tubes garnis d'une feuille enroulée en spirale de ce polymère, toujours comme à l'exemple précédent.

Pour rendre ces tubes biofonctionnels, on a procédé comme suit: on y a fait circuler 2 h une solution d'HCl 2M à 30 °C puis on les a rincés avec cette même solution refroidie à 0 °C. On y a alors introduit et laissé agir 5 min à 0 °C une solution d'HCl 1M et de NaNO₂ 1M; puis on a rincé rapidement les tubes avec un tampon borate, pH 7,5 à 0 °C. On a alors fait circuler 2 h dans les tubes une solution 0,1M de p-aminobenzamidine dans un tampon de borate, pH 8,5, à la cadence de 1 ml/min; puis on a rincé les tubes au tampon borate seul. Par ailleurs, on a préparé, dans une solution 0,02M d'ions Ca + +, un extrait aqueux de pancréas de bœuf comme décrit à l'exemple 11 et on a activé celui-ci par adjonction d'une petite portion de trypsine et lente agitation 3 h à pH 8,5. Il s'est alors formé un précipité qu'on a séparé par centrifugation. On a ensuite prélevé 50 ml du liquide surnageant clair et on l'a fait circuler 1 h dans un jeu de 10 tubes préparés comme décrit ci-dessus, ces tubes étant reliés en série, à la cadence de 2 ml/min. On a lavé les tubes par du tampon 0,1M Tris · HCl, pH 8,1 à 0 °C, puis on y a fait circuler du HCl aqueux dilué, pH 1,7 à 0 °C ce qui a eu pour effet de détacher la trypsine retenue par le revêtement des tubes. Finalement, on a dialysé l'extrait par une membrane standard de cellulose régénérée et on a lyophilisé le concentrat ce qui a fourni 14 mg d'un produit riche en trypsine et correspondant à 64% de la quantité de trypsine totale du liquide centrifugé de départ, telle que mesurée au moyen de BAPNA (cf. exemple 1).

Exemple 6

Dans une plaque de polyéthylène expansé de 2 cm d'épaisseur (mousse à structure ouverte) on a découpé à l'emporte-pièce (perce-bouchon) un cylindre de 4 mm de diamètre. On a imprégné ce «bouchon» de mousse de la composition adhésive de l'exemple 2 puis on l'a introduit dans un tube de quartz de 4 mm de diamètre et on l'a pressé et trituré avec une baguette afin d'en exprimer le plus possible de liquide adhésif. Puis on a relié le tube à une source d'azote comprimé et, tout en faisant circuler ce gaz dans le tube à un rythme très lent, on a irradié le tube 6 min tout en le faisant tourner sur lui-même comme à l'exemple 2.

Après durcissement de l'adhésif on a activé le substrat en faisant circuler dans le tube une solution de trypsine à 100 mg/ml. Après saturation du revêtement par le trypsine, on a fait circuler dans le tube une solution standard de BAPNA (voir exemple 1). Par le dosage de la p-nitroaniline libérée par hydrolyse on a estimé l'activité de la trypsine fixée sur la mousse à 240 µg environ.

Exemple 7

Dans une récipient cylindrique en alumine, on a placé 100 g de billes de verre d'environ 1 mm de diamètre et environ 0,6 g de la composition adhésive de l'exemple 2. On a fait tourner le récipient 2 h sur un mélangeur à rouleaux horizontaux, après quoi on a introduit environ 0,3–0,4 g de ces billes dans un tube de 5 mm de diamètre et d'environ 2 cm de long. Puis on a irradié ce tube 6 min à température ambiante comme décrit aux exemples précédents tout en y faisant passer un lent courant d'azote. Après polymérisation, on a mesuré, par réfractométrie, l'épaisseur du dépôt (phase II) sur les billes et on a constaté que cette épaisseur était de l'ordre de 3 µm. Puis on a procédé à l'activation biofonctionnelle des billes (phase III) comme décrit à l'exemple 2.

Exemple 8

On a sélectionné un tube de polyamide de même type que ceux décrits à l'exemple 2 longueur 5 cm; diamètre 0,15 cm et on l'a revêtu intérieurement d'une couche (phase II) de copolymère au moyen de la composition adhésive de ce même exemple 2.

On a fait circuler 3 h dans ce tube une solution aqueuse contenant les ingrédients suivants: tampon borate 0,5M; pH 8,5; NaCl 0,5M; glucose oxidase 100 mg/ml (MERCK 16 U/mg).

On a placé le tube dans un bain à 25 °C (température stabilisée par thermostat) et à l'aide d'une pompe péristaltique, on a progressivement et successivement fait circuler des échantillons d'1 ml chacun de solution de glucose (dans un tampon phosphate 0,1M à pH 7,0) de concentration croissant, par bonds, de 0,5 à 2,5 g/l; entre le passage de chacun des échantillons de glucose on a intercalé des portions séparatrices de 1 ml de tampon phosphate (0,1M, pH 7,0). Le débit dans le tube était de 3 ml/min. Après passage dans le tube actif, les échantillons ont été recueillis, un à un, dans de petites cuves contenant chacune 0,1 ml de solution aqueuse d'o-dianisidine (0,7 mg/ml) et 0,1 ml de solution de peroxidase (1 mg/ml) dans du tampon phosphate 0,1M, pH 7,0. Après mélange, les solutions des cuves se sont colorées et l'intensité de cette coloration a été mesurée par spectrophotométrie à 436 nm. Les résultats des mesures (densités optiques) sont rassemblés au Tableau I ci-dessous.

Tableau I
Analyse de glucose par la réaction $O_2$ + glucose (glucose-oxydase)→$H_2O_2$ + o-dianisidine (peroxidase)→colorant

| Echantillon | Solution de glucose g/l | Densité optique Lgl/I₀ (436 nm) |
|---|---|---|
| 1 | 0,5 | 0,12 |
| 2 | 1 | 0,242 |
| 3 | 1,5 | 0,363 |
| 4 | 2 | 0,47 |
| 5 | 2,5 | 0,58 |

On remarque donc qu'une relation pratiquement linéaire existe entre la concentration en glucose et la densité optique de la solution de colorant. On peut donc utiliser le revêtement activé suivant l'invention comme élément analytique pour le dosage du glocose dans des solutions aqueuses de concentration inconnue.

Le revêtement enzymatiquement actif est particulièrement stable et son activité se maintient pratiquement sans changement pendant une longue période.

Par ailleurs, on a fait transiter, toujours dans le tube décrit plus haut, des échantillons successifs de 1 ml de solution de glucose, séparés par des portions de 1 ml de tampon phosphate 0,1M, pH 7,0, la concentration de ces échantillons étant, alternativement, de 0,2 et de 2 g/l. On a procédé à la mesure spectrophotométrique des colorations obtenues comme décrit ci-dessus et on a trouvé les résultats ci-dessous.

Tableau II

| Echantillon | Solution de glucose g/l | Densités optiques 436 nm |
|---|---|---|
| 6 | 0,2 | 0,045 |
| 7 | 2,0 | 0,465 |
| 8 | 0,2 | 0,05 |
| 9 | 2,0 | 0,47 |
| 10 | 0,2 | 0,05 |

Les résultats ci-dessous indiquent qu'aucun phénomène important de «mémoire» (carry-over) ne vient troubler la mesure lorsqu'on passe d'une forte concentration en glucose à une concentration 10 fois moindre, et vice-versa. La méthode présente donc des degrés de latitude de concentrations et de fiabilité élevés. De plus, le revêtement enzymatiquement actif est parfaitement stable et son activité se maintient pratiquement sans changement durant une longue période.

Exemple 9

On a placé dans une pipette de 20 ml 21 g de billes de verre de 1 mm recouvertes d'un revêtement photopolymérisé (phase II) telles que décrites à l'exemple 7. Puis on a fait circuler 3 h en continu à température ambiante dans la pipette une solution aqueuse contenant 100 mg/ml de chymotrypsine (SIGMA) dans un tampon phosphate 0,5M, pH 7,5. On a rincé ensuite soigneusement la pipette au tampon phosphate 0,5M puis on a mesuré l'activité spécifique du revêtement ainsi enzymatiquement activé au moyen du p-nitroanilide de la N-glutaryl-L-phénylalanine (GLUPHEPA). Cette méthode, décrite par exemple par B.F. Erlanger et al, dans Arch. Biochim. et Biophys. 115, 206 (1966) est basée sur le clivage du nitroanilide ci-dessus avec libération de p-nitroaniline qu'on mesure spectrophotométriquement à 410 nm. On a ainsi établi que l'activité de la colonne était de 60 µg de chymotrypsine/cm² de revêtement des billes, la surface totale de ces billes était d'environ 300 cm².

Au moyen de la colonne ci-dessus, on a procédé à l'hydrolyse en continu d'une solution à 1% de caséine dans du tampon phosphate 0,1M, pH 7,6. On a fait passer 1 litre de cette solution à travers la colonne ci-dessus à la cadence de 2 ml par min et on a constaté, par analyse de l'hydrolysat suivant les moyens habituels, que le taux de conversion dépassait 90%. Cette analyse a été faite selon la méthode de Kunitz, Methods in Enzymology, Vol. II, p. 33. Pour cela on ajoute de l'acide trichloracétique à un aliquot de solution à doser et, après séparation du précipité formé par centrifugation, on mesure l'absorbance du liquide à 280 nm.

Après avoir laissé reposer la colonne 48 h en présence de tampon phosphate 0,1M, on a, à nouveau, fait passer 1 l d'une solution de caséine à 1% à travers le tube au même débit que précédemment et, par analyse de l'hydrolysat, on a constaté que le pouvoir enzymatique du tube activé était demeuré inchangé.

Exemple 10

On a préparé un film de polyéthylène recouvert d'une couche de composition adhésive comme décrit dans l'exemple 4.

Avant de soumettre le film ainsi couché à la photopolymérisation, on l'a saupoudré d'alumine en poudre de granulométrie environ 5 µm aux taux de 0,5 à 1 g/m². Puis on a irradié le film comme déjà décrit et on l'a découpé en rubans qu'on a enroulés sur eux-mêmes et glissés dans des tubes comme décrit à l'exemple 4. Les grains d'alumine fixés à la surface de l'adhésif font en sorte d'empêcher que les spires jointives du ruban n'adhèrent l'une à l'autre et qu'il subsiste un espace suffisant, entre chaque spire, pour qu'un liquide puisse aisément circuler dans le tube.

On a fait circuler 3 h à température ordinaire dans un des tubes ainsi préparés une solution aqueuse de tampon phosphate 0,5M, pH 7,5, contenant 100 mg/ml de trypsine (SIGMA, type 1X). Puis on a rincé le tube au tampon phosphate et on a déterminé l'activité enzymatique du revêtement fonctionnel au moyen du p-nitroanilide de la benzoyl-arginine (BAPNA) de la manière suivante: on a fait circuler 5 min dans le tube 5 ml de la solution $10^{-3}$M de BAPNA (débit 3 ml/min). Puis on a analysé la solution ainsi traitée par spectrophotométrie à 410 nm (pic d'absorption de la p-nitroaniline). On a ensuite déterminé la grandeur de l'équivalent de trypsine fixée sur le substrat par comparaison avec une courbe d'étalonnage standard, cette courbe représentant le taux de p-nitroaniline en fonction de la quantité de trypsine agissant sur une solution équivalente de BAPNA. Dans le présent cas, on a évalué l'activité du revêtement à environ 30 µg de trypsine par cm².

Exemple 11

Dans un tube en polyamide d'un diamètre de 1,5 mm et de 1 m de long, on a fait circuler une solution à 30% dans du CHCl$_3$ de la composition adhésive de l'exemple 3. On a soigneusement égoutté le tube par gravité puis on y a fait passer un lent courant d'azote chauffé à 40°C. Après 10 min, période au cours de laquelle les traces de CHCl$_3$ ont été évaporées, on a soumis le tube à une irradiation au moyen d'une lampe UV qu'on a déplacée parallèlement au tube, tout en faisant tourner celui-ci sur lui-même, de manière que chaque portion du tube soit soumis au rayonnement de la lampe pendant environ 4 min à une distance de 15 cm.

On a ensuite rempli ce tube, par aspiration, d'une solution à 25% de toluène diisocyanate dans le chloroforme et, après une attente de 15–20 min, on a vidé le tube et on l'a lavé au chloroforme anhydre. Après séchage du tube au moyen d'un courant d'azote sec, on a fait circuler 24 h dans celui-ci une solution à 50 mg/ml d'inhibiteur de soja (FLUKA) dans un tampon borate à pH 9. Puis on a rincé le tube à l'eau distillée. Par ailleurs, on a broyé et homogénéisé dans 600 ml d'eau distillée un pancréas frais de bœuf; on a centrifugé la suspension et fait circuler en continu le liquide surnageant dans le tube activé comme décrit ci-dessus. Les traces de trypsine contenues dans cette solution ont été retenues par le revêtement interne du tube alors que les substances zymogènes (facilement transformées par la trypsine) de l'extrait sont restées inchangées en solution. On a pu ensuite traiter cet extrait, débarrassé de la trypsine indésirable, de manière à en isoler les substances zymogènes de la manière habituelle, celles-ci ayant alors pu être obtenues avec un rendement particulièrement élevé.

**Revendications**

1. Composition photopolymérisable pour revêtement adhésif de résine hydrophile, gonflant à l'eau et perméable à celle-ci, apte à fixer des substances bioactives, comprenant de l'acide acrylique et

(i) au moins une cétone aromatique comme photoinitiateur,

(ii) au moins un acrylate ou méthacrylate d'amino-alcool comme activateur, et

(iii) au moins un monomère acrylique ou vinylique photocopolymérisable comportant, soit des groupes réactifs pour la fixation directe de composés bioactifs, le monomère étant choisi parmi l'acrylate de N-hydroxysuccinimide, l'acrylamido caproate de N-hydroxysuccinimide l'acrylate d'epoxypropanol et l'acrylate de 2-isocyanatoéthyle, soit des chaînons hydrocarbonés intermédiaires pour lier de tels groupes parmi le moyen de fonctions de ces chaînons, celles-ci étant choisies parmi hydrazino-, OH, ester, COOH, NH$_2$, carbodiimide et isocyanate.

2. Composition selon la revendication 1, caractérisée par le fait qu'elle comprend, en outre, au moins un autre composé acrylique choisi parmi les esters acryliques et méthacryliques aliphatiques et cycloaliphatiques, les acrylates polyfonctionnels et les prépolymères acryliques.

3. Composition suivant la revendication 1, caractérisée par le fait qu'elle contient les pourcents pondéraux suivants des divers ingrédients: acide acrylique 10–70; photoinitiateur (i) 1–5; activateur (ii) 0,5–15; monomère photocopolymérisable (iii) jusqu'à 50%.

4. Composition suivant la revendication 2, caractérisée par le fait qu'elle contient, en poids, jusqu'à 60% d'esters acrylique et 5–10% d'acrylates polyfonctionnels ou prépolymères.

5. Revêtement photopolymérisé hydrophile adhérant aux supports organiques et minéraux constitué d'une résine acrylique contenant, à l'état copolymérisé, au moins un maillon provenant d'un monomère acrylique ou vinylique comportant, soit un groupe réactif apte à fixer directement des composés bioactifs, le monomère étant choisi parmi l'acrylate de N-hydroxysuccinimide, l'acrylamidocaproate de N-hydroxysuccinimide, l'acrylate d'epoxypropanol et l'acrylate de 2-isocyanato-éthyle, soit des chaînons intermédiaires pour lier de tels groupes par le moyen de fonctions de ces chaînons choisies parmi OH, ester, COOH, NH$_2$, carbodiimide et isocyanate.

6. Revêtement suivant la revendication 5, caractérisé par le fait qu'il comprend, fixées directement ou indirectement à l'aide dudit groupement réactif, une ou plusieurs substances choisies parmi les enzymes, inhibiteurs d'enzymes, antigènes, anticorps, lectines, ferments, microbes, hormones, héparine et autres macro-molécules biofonctionnelles à l'état actif ou temporairement inhibé.

7. Substrat en matière organique ou minérale naturelle ou synthétique dont la surface possède, au moins en partie, une activité biofonctionnelle, caractérisé par le fait que cette surface est recouverte d'un revêtement suivant la revendication 6.

8. Substrat en matière organique ou minérale naturelle ou synthétique dont la surface est recouverte, au moins en partie, d'un revêtement adhésif obtenu par photopolymérisation de la composition suivant la revendication 1.

9. Substrat dont la surface est recouverte, au moins en partie, d'un revêtement adhésif résultant de la photopolymérisation de la composition suivant la revendication 1 et, ultérieurement, de la fixation sur ce revêtement d'une ou plusieurs substances biofonctionnelles.

10. Procédé pour la préparation d'un revêtement suivant la revendication 5, caractérisé par le fait qu'on prépare une composition adhésive suivant la revendication 1, qu'on étale celle-ci sur un substrat minéral ou organique et qu'on photopolymérise le film ainsi obtenu.

11. Procédé pour la préparation d'un revêtement suivant la revendication 6, caractérisé par le fait qu'on prépare une composition adhésive suivant la revendication 1, qu'on étale celle-ci sur un substrat minéral ou organique de manière à former un film mince sur la surface de ce substrat, qu'on irradie celui-ci au moyen d'une source actinique afin de photopolymériser ce film, puis qu'on met en contact le substrat avec une substance biofonctionnelle, cette dernière se fixant sur ledit revêtement.

12. Procédé suivant la revendication 10, caractérisé par le fait qu'on imprègne de composition une mousse transparente aux rayons UV de manière à obtenir une structure poreuse dont la surface interne est recouverte d'un film qu'on durcit par irradiation.

13. Procédé suivant la revendication 10, caractérisé par le fait que le substrat est un film de polyéthylène très mince qu'on enroule ou replie ensuite de façon à ce qu'il occupe un volume réduit.

14. Procédé suivant la revendication 10, caractérisé par le fait que le grammage du film de composition est de 0,1 à 5 g/m².

15. Procédé suivant la revendication 10, caractérisé par le fait que le substrat est un tube, le revêtement étant appliqué sur la surface interne de celui-ci, ce tube servant ensuite d'élément d'analyse de constituants de fluides biologiques.

16. Utilisation des substrats suivant les revendications 7 ou 9 comme éléments d'analyse automatique de constituants de fluides biologiques.

17. Utilisation des substrats suivant les revendications 7 ou 9 comme biocatalyseurs de réactions biochimiques, pour éliminer, par extraction, des molécules biofonctionnelles indésirables dans des solutions bioactives ou pour la purification, par extraction sélective dans des solutions d'extraits de matières organiques, de molécules bioactives.

## Claims

1. Photopolymerizable composition providing an adhesive coating of water swellable hydrophilic resin permeable to water and capable of binding bioactive substances, comprising acrylic acid and

(i) at least one aromatic ketone as photoinitiator

(ii) at least one aminoalcohol acrylate or methacrylate as activator, and

(iii) at least a photopolymerizable acrylic or vinylic monomer comprising either reactive groups for the direct binding of bioactive compounds, the monomer being selected from N-hydroxysuccinimide acrylate, N-hydroxysuccinimide acrylamidocaproate, epoxypropanol acrylate, and 2-isocyanatoethyl acrylate, or intermediate hydrocarbon links for binding such groups by means of functions of said links, said functions being selected from hydrazino-, OH, ester, −COOH, −NH$_2$, carbodiimide and isocyanate.

2. Composition according to claim 1, characterized in that it further comprises at least another acrylic compound selected from aliphatic and cycloaliphatic acrylic and methacrylic esters, polyfunctional acrylates and acrylic prepolymers.

3. Composition according to claim 1, characterized in that it contains the various ingredients in the following percent by weight: acrylic acid 10–70; photoinitiator (i) 1–5; activator (ii) 0.5–15; photocopolymerizable monomer (iii) up to 50%.

4. Composition according to claim 2, characterized in that it contains, by weight, up to 60% of acrylic esters and 5–10% of prepolymers or polyfunctional acrylates.

5. Photopolymerized hydrophilic coating adhering to organic and mineral substrates constituted from an acrylic resin containing, in the polymerized state, at least a link from an acrylic or vinyl monomer comprising either a reactive group capable of binding directly bioactive compounds, the monomer being selected from N-hydroxysuccinimide acrylate, N-hydroxysuccinimide acrylamidocaproate, epoxypropanol acrylate and 2-isocyanatoethyl acrylate, or intermediate links for binding such groups by means of functions of such links selected from OH, ester, COOH, NH$_2$, carbodiimide and isocyanate.

6. Coating according to claim 5, characterized in that it comprises, directly or indirectly bound by means of said reactive grouping, one or more substances selected from enzymes, enzyme inhibitors, antigens, antibodies, lectins, ferments, microbes, hormones, heparin and other biofunctional macro-molecules in the active state or temporarily inactivated.

7. Substrate of natural or synthetic organic or mineral material the surface of which possesses, at least in part, a biofunctional activity, characterized in that this surface is covered with a coating according to claim 6.

8. Substrate made of an organic or mineral natural or synthetic material the surface of which is covered, at least partly, with an adhesive coating obtained by photopolymerizing the composition according to claim 1.

9. Substrate the surface of which is covered, at least partly, with an adhesive coating resulting from the photopolymerization of the coating according to claim 1 and, subsequently, from the binding to said coating of one or several biofunctional substances.

10. Process for the preparation of a coating according to claim 5, charactarized in that there is prepared an adhesive composition according to claim 1, the latter is spread on a mineral or organic carrier substrate and the film thus obtained is photopolymerized.

11. Process for the preparation of a coating according to claim 6, characterized in that there is prepared an adhesive composition according to claim 1, the latter is spread on a mineral or organic carrier substrate so as to form a thin film on the surface of said substrate, the latter is irradiated with an actinic source so as to photopolymerize said film, then the substrate is contacted with a biofunctional substance, the latter becoming bound to said coating.

12. Process according to claim 10, characterized in that a UV transparent foam is impregnated with the composition so as to obtain a porous structure of which the internal surface is coated with a film to be hardened by irradiation.

13. Process according to claim 10, characterized in that the substrate is a very thin polyethylene film which is thereafter wound or folded so that it is reduced to a small volume.

14. Process according to claim 10, characterized in that the surface weight of the film of composition is 0.1 to 5 g/m².

15. Process according to claim 10, characterized in that the substrate is a tube, the coating being applied to the internal surface thereof, the tube being thereafter used as an analytical element for the constituents of biological fluids.

16. The use of substrates according to claim 7 or 9 as elements for the automatic analysis of constituents of biological fluids.

17. The use of the substrates according to claim 7 or 9 as biocatalysts in biochemical reactions for eliminating, by extraction, undesirable biofunctional molecules from bioactive solutions or for the purification, by selective extraction from extract solutions of organic materials, of bioactive molecules.

## Patentansprüche

1. Photopolymerisierbare Zusammensetzung für einen adhäsiven Überzug aus hydrophilem Harz, der in Wasser aufquillt und für dieses durchlässig ist, sowie an bioaktiven Substanzen befestigt werden kann, welche Acrylsäure und

(i) wenigstens ein aromatisches Keton als Photoinitiator,

(ii) wenigstens ein Aminoalkoholacrylat oder -methacrylat als Aktivator und

(iii) wenigstens ein photopolymerisierbares Acryl- oder Vinylmonomer enthält, das entweder reaktive Gruppen für die direkte Befestigung bioaktiver Verbindungen, wobei das Monomer aus dem Acrylat von N-Hydroxysuccinimid, dem Acrylamidocaproat von N-Hydroxysuccinimid, dem Acrylat von Epoxypropanol und dem Acrylat von 2-Isocyanatoäthyl ausgewählt wird, oder Kohlenwasserstoffzwischenketten aufweist, um derartige Gruppen mittels der Funktionen dieser Ketten zu verbinden, die aus Hydrazino-, OH, Ester, COOH, NH₂, Carbodiimid und Isocyanat ausgewählt sind.

2. Zusammensetzung nach Anspruch 1, dadurch gekennzeichnet, dass sie überdies wenigstens eine weitere Acrylverbindung umfasst, die aus den aliphatischen und cycloaliphatischen Acryl- und Methacrylestern, den polyfunktionellen Acrylaten und den Acrylvorpolymeren ausgewählt ist.

3. Zusammensetzung nach Anspruch 1, dadurch gekennzeichnet, dass sie die folgenden Gewichtsprozente an verschiedenen Bestandteilen enthält: Acrylsäure 10–70; Photoinitiator (i) 1–5; Aktivator (ii) 0,5–15; photocopolymerisierbares Monomer (iii) bis 50%.

4. Zusammensetzung nach Anspruch 2, dadurch gekennzeichnet, dass sie bis 60 Gew.-% Acrylsäureester und 5–10 Gew.-% polyfunktioneller Acrylate oder Vorpolymere enthält.

5. Hydrophiler photopolymerisierbarer Überzug, der an organischen oder mineralischen Trägern haftet und aus einem Acrylharz besteht, das im copolymerisierten Zustand wenigstens ein Kettenglied besitzt, das von einem Acryl- oder Vinylmonomer herrührt, und zwar entweder eine reaktive Gruppe, die direkt auf den bioaktiven Verbindungen befestigt werden kann, wobei das Monomer aus dem Acrylat von N-Hydroxysuccinimid, dem Acrylamidocaproat von N-Hydroxysuccinimid, dem Acrylat von Epoxypropanol und dem Acrylat von 2-Isocyanato-äthyl ausgewählt ist, oder Zwischenketten aufweist, um derartige Gruppen mit Hilfe der Funktionen dieser Gruppen zu verbinden, die aus OH, Ester, COOH, NH₂, Carbodiimid und Isocyanat ausgewählt sind.

6. Überzug nach Anspruch 5, dadurch gekennzeichnet, dass er, mit Hilfe dieser reaktiven Gruppierung direkt oder indirekt befestigt, eine oder mehrere Substanzen umfasst, die aus den Enzymen, Enzyminhibitoren, Antigenen, Antikörpern, Lectinen, Fermenten, Mikroben, Hormonen, Heparin oder anderen biofunktionellen Makromolekülen im aktiven oder zeitlich gehemmten Zustand ausgewählt sind.

7. Substrat aus organischem oder natürlichem oder synthetischem, mineralischem Material, dessen Oberfläche wenigstens zum Teil eine biofunktionelle Aktivität besitzt, dadurch gekennzeichnet, dass diese Oberfläche mit einem Überzug gemäss Anspruch 6 überzogen ist.

8. Substrat aus organischem oder natürlichem oder synthetischem, mineralischem Material, dessen Oberfläche wenigstens zum Teil mit einem adhäsiven Überzug versehen ist, der durch Photopolymerisation der Zusammensetzung nach Anspruch 1 erhalten wird.

9. Substrat, dessen Oberfläche wenigstens zum Teil von einem adhäsiven Überzug überzogen ist, der sich aus der Photopolymerisation der Zusammensetzung nach Anspruch 1 ergibt und wobei eine oder mehrere biofunktionelle Substanzen schliesslich auf diesem Überzug befestigt werden.

10. Verfahren zur Herstellung eines Überzuges gemäss Anspruch 5, dadurch gekennzeichnet, dass man eine adhäsive Zusammensetzung gemäss Anspruch 1 herstellt, dass man diese auf einem mineralischen oder organischen Substrat ausbreitet und dass man den so erhaltenen Film photopolymerisiert.

11. Verfahren zur Herstellung eines Überzuges gemäss Anspruch 6, dadurch gekennzeichnet, dass man eine adhäsive Zusammensetzung gemäss Anspruch 1 herstellt, dass man diese auf einem mineralischen oder organischen Substrat derart verteilt, dass ein dünner Film auf der Oberfläche dieses Substrates gebildet wird, dass man diesen mittels einer aktinischen Quelle bestrahlt, um diesen Film zu photopolymerisieren, dass man dann das Substrat mit einer biofunktionellen Substanz in Kontakt bringt, wobei sich diese auf dem Überzug befestigt.

12. Verfahren nach Anspruch 10, dadurch gekennzeichnet, dass man einen Schaum mit der Zusammensetzung imprägniert, der für UV-Strahlen durchlässig ist, so dass man eine poröse Struktur erhält, dessen innere Oberfläche von einem Film überzogen ist, den man durch Bestrahlung härtet.

15

13. Verfahren nach Anspruch 10, dadurch gekennzeichnet, dass das Substrat ein sehr dünner Polyäthylenfilm ist, den man dann einrollt oder faltet, derart, dass dieser ein verringertes Volumen einnimmt.

14. Verfahren nach Anspruch 10, dadurch gekennzeichnet, dass das Quadratmeter-Gewicht des Filmes der Zusammensetzung 0,1 bis 5 g/m² beträgt.

15. Verfahren nach Anspruch 10, dadurch gekennzeichnet, dass das Substrat ein Rohr ist, wobei der Überzug auf der inneren Fläche angeordnet ist und dieses Rohr dann als Analyseelement für Bestandteile biologischer Medien dient.

16. Verwendung der Substrate nach den Ansprüchen 7 oder 9, als automatische Analyseelemente für Bestandteile biologischer Medien.

17. Verwendung der Substrate nach den Ansprüchen 7 oder 9, als Biokatalysator für biochemische Reaktionen, um durch Extraktion unerwünschte biofunktionelle Moleküle in bioaktiven Lösungen zu eliminieren oder um durch selektive Extraktion in Lösungen von Extrakten organischen Materials bioaktive Moleküle zu reinigen.